# EUROPÄISCHE PATENTSCHRIFT

(11) **EP 3 753 634 B1**
(45) Veröffentlichungstag und Bekanntmachung des Hinweises auf die Patenterteilung: **04.08.2021**
(21) Anmeldenummer: 19181249.4
(22) Anmeldetag: 19.06.2019
(51) Int. Cl.: B01L 3/00, G01N 1/28

(54) **PROBENTRÄGER ZUR AUFNAHME VON GETROCKNETEN BLUTBESTANDTEILEN UND VERFAHREN ZUR GEWINNUNG EINES MEMBRANELEMENTES MIT GETROCKNETEN BLUTBESTANDTEILEN**
SAMPLE CARRIER FOR RECEIVING DRIED BLOOD COMPONENTS AND METHOD FOR OBTAINING A MEMBRANE ELEMENT WITH DRIED BLOOD COMPONENTS
SUPPORT D'ÉCHANTILLON PERMETTANT DE RECEVOIR DES COMPOSANTS SANGUINS SÉCHÉS ET PROCÉDÉ DE RÉCUPÉRATION D'UN ÉLÉMENT MEMBRANE À L'AIDE DES COMPOSANTS SANGUINS SÉCHÉS

(43) Veröffentlichungstag der Anmeldung: 23.12.2020
(73) Patentinhaber: Euroimmun Medizinische Labordiagnostika AG, 23560 Lübeck (DE)
(72) Erfinder: WEIMANN, Alf, 23560 Lübeck (DE); SEISMANN, Henning, 23863 Nienwohld (DE); HONOLD, Lisa, 71126 Gäufelden (DE); STÖCKER, Winfried, 23627 Gross Grönau (DE)

(56) Entgegenhaltungen:
- WO-A1-2012/130457
- WO-A1-2013/006904
- WO-A1-2016/156376
- WO-A2-2009/095826
- FUMIN LI ET AL: "Perforated dried blood spots: a novel format for accurate microsampling", BIOANALYSIS, Bd. 3, Nr. 20, 1. Oktober 2011 (2011-10-01), Seiten 2321-2333, XP055147130, ISSN: 1757-6180, DOI: 10.4155/bio.11.219

## Beschreibung

Die Erfindung betrifft einen Probenträger. Der Probenträger weist eine absorptionsfähige Membranschicht auf, welche auf einem nicht gewobenen Polyolefin basiert und welche wenigstens einen Aufnahmebereich mit getrockneten Blutbestandteilen aufweist. Ferner weist er eine Folienschicht basierend auf einem Kunststoff auf, wobei die Folienschicht antistatisch vorbehandelt ist. Die Membranschicht und die Folienschicht sind in einem Teilbereich des Probenträgers miteinander mechanisch verbunden, wobei die Schichten ferner im Aufnahmebereich nicht miteinander mechanisch verbunden sind. Vorzugsweise bedeckt die Membranschicht die Folienschicht zumindest in dem Aufnahmebereich bei einem Auflegen des Probenträgers auf eine ebene Fläche.

Die Erfindung betrifft ferner ein Verfahren zur Gewinnung eines Membranelementes mit getrockneten Blutbestandteilen. Für das Verfahren wird ein Probenträger bereitgestellt, welcher auf einen Auflagebereich aufgelegt wird. Unterhalb des Auflagebereiches wird ein Gefäß bereitgestellt. Das Membranelement wird aus dem Aufnahmebereich des Probenträgers mittels einer Stanzvorrichtung ausgestanzt, sodass das ausgestanzte Membranelement und ein aus der Folienschicht ausgestanztes Folienelement in das Gefäß fallen.

Für viele qualitative Bestimmungen auf dem Gebiet der Analytik und speziell der Labordiagnostik kommt es nur darauf an, dass die Anwesenheit oder Abwesenheit eines Analyten richtig festgestellt werden kann, nicht jedoch in welcher Konzentration er im Blut eines Patienten vorliegt. Das ist z.B. der Fall, wenn die Blutprobe darauf untersucht wird, ob der Patient an einer Stoffwechselkrankheit leidet, die mit einem defekten Gen assoziiert ist. Die Blutprobe muss dann lediglich darauf untersucht werden, ob ein solches Gen *vorhanden* ist, nicht jedoch wie viel von dem genetischen Material umfassend das Gen in der Probe enthalten ist.

Im Falle anderer Analyten muss festgestellt werden, in welcher Konzentration der Analyt im Blut auftritt, weil ein diagnostischer Referenz-Konzentrationsbereich bekannt ist. Liegt die Konzentration des Analyten in diesem Bereich, so spricht dies dafür, dass der Patient gesund ist. Für solche semi-quantitativen oder auch rein quantitativen Bestimmungen kommt es daher darauf an, dass die Menge oder Konzentration des Analyten richtig bestimmt wird.

Für die Messung von Konzentrationen bestimmter Analyten im Blut von Patienten wird dem Patient meistens venöses Blut abgenommen, gefolgt von der Aufbereitung des Blutes zu Serum. Abgesehen davon, dass dieser invasive Eingriff für den Patienten unangenehm ist und in geringem Maße sogar gesundheitliche Risiken birgt, wie das der Übelkeit oder der Ohnmacht, kann die Blutabnahme nur durch eine qualifizierte Fachkraft wie einen Arzt oder wenigstens eine Krankenschwester oder erfahrene Laborantin durchgeführt werden. Der Patient muss dazu eine Arztpraxis oder ein Krankenhaus aufsuchen.

Viel einfacher und schonender ist hingegen die Gewinnung von Kapillarblut. Nach dem Einstechen mit einem spitzen Gegenstand in die Fingerkuppe oder das Ohrläppchen des Patienten treten einige Tropfen Kapillarblut aus, die mit einer Pipette aufgenommen und auf einen absorptionsfähigen Probenträger gegeben werden. Sobald das Blut auf dem Träger eingetrocknet ist, ggf. bei Raumtemperatur vollständig nach einigen Stunden, kann der Träger ohne weitere Bearbeitung transportiert werden, sehr einfach sogar per Briefpost. Der Besuch der Arztpraxis wird damit überflüssig. Aufgrund der getrockneten Blutprobe bzw. des getrockneten Blutbestandteils gilt der Probenträger mit der darauf befindlichen Probe nicht mehr als Gefahrgut. Aus einem Träger mit eingetrocknetem Blut, bevorzugt Kapillarblut, kann ein Stück Träger mit Blut abgelöst werden. Das kann beispielsweise mit einer Vorrichtung, wie sie in der US14/900,360 beschrieben ist, oder durch Ausstanzen erreicht werden. Das abgelöste Stück Träger mit eingetrocknetem Blut wird häufig als "Dried Blood Spot" oder "DBS" bezeichnet.

Ein hier genannter Träger kann beispielsweise durch eine Membranschicht gegeben sein, welche das Blut bzw. Blutbestandteile in einem flüssigen Aggregatzustand aufnehmen kann und auf dem dann eben das Blut bzw. Blutbestandteile eintrocknen bevor der Träger in Form der absorptionsfähigen Membran transportiert wird.

Bei dem von der Membran aufgenommenen Blutbestandteil kann es sich um Vollblut handeln, insbesondere Kapillarblut. Alternativ handelt es sich um Blutserum oder Blutplasma.

Wird aus der Membran in der zuvor erwähnten Weise dann ein Membranelement bzw. ein Stück des Trägers ausgestanzt, so kann es hierbei vorkommen, dass das Stanzen nicht erfolgreich durchgeführt werden kann.

Üblicherweise wird der Probenträger in Form der Membran auf einer Auflagevorrichtung bzw. einen Auflagebereich aufgelegt und dann mittels einer Stanzvorrichtung bzw. eines Stanzmessers in der Weise mechanisch von oben her auf die Membran eingewirkt, dass ein Membranelement aus der Membran ausgeschnitten wird, wobei es erwünscht ist, dass das Membranelement dann in ein unterhalb des Probenträgers bzw. unterhalb der Membran positioniertes Gefäß aufgrund der Schwerkraft hineinfällt. Dieses Gefäß kann dann später für eine biochemische Analyse verwendet werden.

Da die Membran bzw. die Membranschicht eine gewisse mechanische Flexibilität aufweist, kann es vorkommen, dass während des Stanzvorgangs die Membranschicht zu einem gewissen Maße dem Stanzmesser nachgibt, sodass nicht wirklich ein sauberer Schnitt zum Ausschneiden des Membranelementes erfolgt, sondern dass es in dem Brandbereich des Membranelementes bzw. einer Schnittkante zu einem Einreißen des Membranstoffs kommt. Hierdurch kann es möglich sein, dass Aufgrund des Einreißvorganges zusätzliche Kräfte auf das Membranelement wirken, sodass das Membranelement nicht senkrecht hinunter fällt, sondern in seiner Fallrichtung ausgelenkt wird. Eine statische Aufladung und ein Auslenken des Membranelementes durch elektrostatische Kräfte wird durch die antistatische Folie reduziert und ggf. vermieden. Hierbei kann es vorkommen, dass das Membranelement nicht in das gewünschte und bereitgestellte Gefäß hineinfällt.

Wird die Membranschicht zu dick bzw. zu stark dimensioniert, so kann es vorkommen, dass das Stanzmesser diese Membranschicht nicht vollständig und erfolgreich durchdringt, sodass das Membranelement nicht vollständig aus der Membranschicht ausgeschnitten wird. Auch dann ist eben ein Hineinfallen des Membranelementes in das bereitgestellte Gefäß nicht erreicht worden.

Bei Aufbringen des Blutbestandteiles in einem flüssigen Aggregatzustand auf den Aufnahmebereich der Membranschicht kann es gewünscht sein, dass diese Membranschicht auf ihrer Oberseite als auf ihrer Unterseite mit anderen Schichten nicht in direktem Kontakt steht, um eine Verteilung des flüssigen Blutbestandteils in der Membranschicht bzw. weg von der Membranschicht nicht zu beeinflussen. Hierfür ist es vorteilhaft, dass die Membranschicht und die Folienschicht in dem Aufnahmebereich eben nicht miteinander mechanisch verbunden sind, sodass die Folienschicht für eine Zeit des Trocknungsvorgangs des Blutbestandteils in dem Aufnahmebereich von der Membranschicht weggelenkt werden kann.

Dadurch, dass die Folienschicht jedoch mit der Membranschicht in einem Teilbereich, vorzugsweise einem Randbereich, des Probenträgers mechanisch verbunden ist, wird gewährleistet, dass die Folienschicht auch später für den Stanzvorgang in einfacher Weise bereitgestellt werden kann. Ein Anwender müsste dann also lediglich die Folienschicht von der Membranschicht wegblenden für die Zeit des Trocknungsvorgangs.

Bezogen auf den Probenträger kann die Membranschicht auch als die Oberseite bzw. obere Schicht des Probenträgers bezeichnet werden und die Folienschicht als die Unterseite bzw. die untere Schicht des Probenträgers.

Probenträgerkarten zur Aufnahme von getrockneten Blutbestandteilen, die eine Absorptionsschicht und eine Folienschicht aus Kunststoff aufweisen, sind aus den Druckschriften WO 2013/006904 A1, WO 2016/156376 A1, WO 2012/130457 A1 und WO 2009/095826 A2 bekannt.

Aufgabe der vorliegenden Erfindung ist es, einen Probenträger bereitzustellen, welcher bei Durchführung eines aus dem Stand der Technik bekannten Stanzverfahrens ein erfolgreiches Ausstanzen eines Membranelementes, welches einen getrockneten Blutbestandteil aufweist, so ermöglicht, dass das ausgestanzte Membranelement in ein bereitgestelltes und insbesondere unterhalb der Membranschicht befindliches Gefäßes hineinfällt.

Diese Aufgabe wird gelöst durch einen erfindungsgemäßen Probenträger. Dieser weist eine absorptionsfähige Membranschicht auf, welche auf einem nicht gewobenen Polyolefin basiert und welche wenigstens einen Aufnahmebereich mit getrockneten Blutbestandteilen aufweist. Ferner weist er eine Folienschicht basierend auf einem Kunststoff auf, wobei die Folienschicht antistatisch vorbehandelt ist. Die Membranschicht und die Folienschicht sind in einem Teilbereich des Probenträgers miteinander mechanisch verbunden, wobei die Schichten ferner in dem Aufnahmebereich nicht miteinander mechanisch verbunden sind. Vorzugsweise bedeckt die Membranschicht die Folienschicht zumindest in dem Aufnahmebereich beim Auflegen des Probenträgers auf eine ebene Fläche.

Ein Vorteil des erfindungsgemäßen Probenträgers liegt darin, dass Aufgrund der zusätzlich zur Membranschicht vorgesehenen Folienschicht eine mechanische Stabilisierung der Membranschicht während des Stanzvorgangs ermöglicht wird, sodass ein Nachgeben der Membranschicht während des Stanzens bzw. Schneidevorgangs reduziert wird. Hierdurch kann es gegebenenfalls erreicht werden, dass der Membranstoff im Bereich der Stanzkante bzw. der Schnittkante nicht ausfranst und somit ein Ausschneiden des Membranelementes aus der Membranschicht präziser erreicht werden kann. Durch ein Vermeiden von Ausfransungen im Bereich der Schnittkante bzw. Stanzkante wird es also verhindert, dass das ausgestanzte Membranelement in eine falsche Richtung gelenkt wird und ggf. nicht in ein unterhalb der Stanzvorrichtung positioniertes Gefäß herabfällt.

Dadurch, dass die Membranschicht und die Folienschicht in dem ausgestanzten Aufnahmebereich nicht miteinander mechanisch verbunden sind, wird es ferner erreicht, dass das ausgestanzte Membranelement nach Hineinfallen in das bereitzustellende Gefäß mit seiner gesamten Oberfläche, also auf seiner Rückseite bzw. die der Folienschicht zugewandten Seite, mit Reagenzien bzw. biochemikalischen Flüssigkeiten in dem Gefäß in Kontakt kommen kann, sodass zu erwartende Prozessionsergebnisse nicht verfälscht werden.

Dadurch, dass die Folienschicht jedoch in einem Teilbereich des Probenträgers, vorzugsweise einem Randbereich des Probenträgers, mit der Membranschicht mechanisch verbunden ist, ergibt sich eine einfache Handhabbarkeit für den Anwender. Der Anwender muss also den Probenträger nur an einer bestimmten Stelle, beispielsweise dem hier genannten Teilbereich bzw. Randbereich, anfassen und kann dann in einem einzigen Arbeitsschritt sowohl die Folienschicht als auch die Membranschicht gemeinsam auf einem Auflagebereich der Stanzvorrichtung positionieren, sodass Membranschicht und Folienschicht in einer gewünschten Weise zueinander angeordnet bzw. ausgerichtet sind. Es ist also nicht erforderlich, dass der Anwender erst eine separate Folienschicht auflegt und dann die Membranschicht auf die Folienschicht auflegt. Der Probenträger kann zur Aufnahme von Blutbestandteilen in einem flüssigen Aggregatzustand in einem Labor, einer Arztpraxis oder auch an einem Wohnort eines Patienten verwendet werden und dann nach trocknen der Blutbestandteile verschickt bzw. zu einem Laborarbeitsplatz gebracht werden, ohne dass dort dann eine separate Folienschicht bereitgestellt werden muss.

Dadurch, dass die Folienschicht antistatisch vorbehandelt ist, wird ein Entstehen von elektrostatischer Aufladung bzw. elektrostatischen Kräften während des Stanzvorgangs vermieden. Da die Membranschicht und die Folienschicht aufeinander liegen, durchschneidet während des Stanzvorganges das Stanzmesser sowohl Membranschicht als auch die Folienschicht. Ein solches Stanzmessers ist üblicherweise aus Metall. Ein Reiben von Metall an einem Kunststoff während des Stanzvorganges kann eine elektrostatische Aufladung der Folie und ggf. auch des Membranelementes bewirken, so dass elektrostatische Kräfte auf das ausgestanzte Folienelement und ggf. auch das Membranelement wirken können. Hierdurch könnte es dazu kommen, dass das ausgestanzte Folienelement und ggf. auch das Membranelement in seiner Fallrichtung abgelenkt wird und nicht in das positionierte Gefäß hineinfällt. Würde zum Beispiel das ausgestanzte Folienelement in ein anderes, neben dem gewünschten Gefäß befindliches, anderes Gefäß hineinfallen, so könnte es bei Durchführung mehrerer Ausstanzvorgänge für mehrere Aufnahmebereiche eines Probenträgers zu einer Kreuzkontamination zwischen unterschiedlichen Blutbestandteilen bzw. unterschiedlichen Blutproben in den jeweiligen Gefäßen kommen. Dadurch, dass die Folienschicht antistatisch vorbehandelt ist, wird es vermieden, dass elektrostatische Kräfte eine unerwünschte Fehllenkung des ausgestanzten Membranelementes und/oder des ausgestanzten Folienelementes bewirken können.

Vorzugsweise ist die Folienschicht beidseitig antistatisch vorbehandelt.

Unter dem Begriff "quantitative Bestimmung" wird im Sinne dieser Anmeldung eine Bestimmung verstanden, die eine Aussage über die absolute Konzentration des Analyten gestattet, noch bevorzugter mit einem numerischen Wert. Es kann sich alternativ um eine semi-quantitative Bestimmung handeln, bei der eine Zuordnung der Konzentration zu einem Bereich aus wenigstens drei, möglichst vier Konzentrationsbereichen ermöglicht wird, z.B. negativ, schwach positiv und positiv, oder eine relative Konzentrationsbestimmung. Insbesondere erfolgt die Konzentrationsbestimmung mit Hilfe von Kalibratoren, wobei es sich bevorzugt um zwei oder mehr, bevorzugt vier Einheiten, bevorzugt Lösungen oder auf einem diagnostisch nützlichen Träger gecoateten festen Analyten handelt, die jeweils eine bekannte Menge des Analyten enthalten, wobei die zwei oder mehr Einheiten jeweils eine andere bekannte Menge umfassen.

Eine quantitative Bestimmung wird vorzugsweise mit einem Verfahren ausgeführt, das aus der Gruppe ausgewählt ist, die Immundiffusion, Immunelektrophorese, Lichtstreuung, Agglutination und Immuntest mit Markierung - wie die aus der Gruppe umfassend Immuntest mit radioaktiver Markierung, mit enzymatischer Markierung, bevorzugter ELISA, mit Chemilumineszenz-Markierung, bevorzugter Elektrochemilumineszenz-Markierung und mit Immunfluoreszenz-Markierung, bevorzugter indirekte Immunfluoreszenz-Markierung - umfasst, bevorzugt mit ELISA.

Der getrocknete Blutbestandteil wird aus dem ausgetanzten Membranelement durch Kontaktieren des ausgetanzten Membranelementes mit einer Flüssigkeit eluiert, die geeignet ist, um den Analyten aus dem eingetrockneten Blutbestandteil aufzunehmen. Dabei kommen insbesondere wässrige Puffer mit geeignetem pH und Salzgehalt in Frage, beispielsweise PBS. Die genaue Zusammensetzung der Flüssigkeit und die Bedingungen und die Dauer des Kontaktierens können durch Routineversuche zur Stabilisierung und Optimierung zwecks möglichst vollständiger Aufnahme des Analyten in die Flüssigkeit herausgefunden werden und hängen von der Natur des Analyten ab. Die Flüssigkeit wird möglichst auch gleich derart gewählt, dass sie mit dem nachfolgend eingesetzten Verfahren zum Nachweisen des Analyten kompatibel ist. Anschließend wird der Analyt von Interesse in der Flüssigkeit nachgewiesen. Dabei wird bestimmt, ob der Analyt anwesend oder abwesend bzw. in einer Konzentration oberhalb der Nachweisgrenze der eingesetzten Nachweismethode vorhanden ist. Bevorzugt wird der Analyt semi-quantitativ oder quantitativ nachgewiesen. Verschiedene Optionen zur Durchführung des Verfahrens sind im Stand der Technik beschrieben, z.B. Grüner, N., Stambouli, O. und Ross, R. S. (2015) Dried Blood Spots - Preparing and Processing for Use in Immunoassays and in Molecular Techniques, J. Vis. Exp 97, 52619.

Vorteilhafte Ausführungsformen der Erfindung sind Gegenstand der abhängigen Ansprüche und werden in der folgenden Beschreibung unter teilweiser Bezugnahme auf die Figuren näher erläutert.

Vorzugsweise ist der Kunststoff ein Polyester.

Vorzugsweise ist der Polyester Polyethylenterephthalat.

Vorzugsweise ist die Folienschicht in dem Sinne antistatisch vorbehandelt, dass die Folienschicht zumindest in einem Teilbereich ihrer Oberfläche ein Antistatikum aufweist. Vorzugsweise weist die Folienschicht auf beiden Seiten jeweils in zumindest einem Teilbereich ein Antistatikum auf.

Vorzugsweise ist das Polyolefin aus einer Gruppe umfassend Polyethylen, Polypropylen, Polymethylpente, Polyisobutylen und Polybutylen ausgewählt.

Vorzugsweise ist das Polyolefin Polypropylen.

Vorzugsweise weist der Probenträger wenigstens ein verstellbares Aufstellelement auf, welches zwischen einer ersten Konfiguration und der zweiten Konfiguration verstellbar ist. In der ersten Konfiguration ermöglicht das Aufstellelement bei Auflegen des Probenträgers auf eine ebene Fläche eine Beabstandung der Membranschicht von der Folienschicht. In der zweiten Konfiguration bedeckt bei Auflegen des Probenträgers auf die ebene Fläche die Membranschicht zumindest in dem Aufnahmebereich die Folienschicht.

Wird der Blutbestandteil in einem flüssigen Aggregatzustand auf den Aufnahmebereich des Probenträger aufgebracht, so kann durch dieses Aufstellelement in der ersten Konfiguration eine Beabstandung der Folienschicht von der Membranschicht bewirkt werden, sodass für eine Zeit eines Trocknungsvorgangs der Blutbestandteil nicht aus der Membranschicht bzw. aus dem Aufnahmebereich der Membranschicht auf die Folienschicht gelangt und dort weg von dem Aufnahmebereich bzw. weg von der Membranschicht geführt wird. Hierdurch wird eine Maximierung eines Verweilens des zunächst flüssigen Blutbestandteils in dem Aufnahmebereich bzw. in der Membranschicht für die Zeit des Trocknungsvorganges maximiert. Mit anderen Worten: der Blutbestandteil gelang nicht auf die Folienschicht, sodass möglichst viel flüssiger Blutbestandteil in der Membranschicht während des Trockenvorganges verbleibt. Dies ist insbesondere deshalb vorteilhaft, da der Blutbestandteil als eine Patientenprobe häufig nur in begrenzter Maße bzw. mit einem begrenzten Volumen bereitgestellt werden kann daher eine kostbare Ressource darstellt.

Dadurch, dass in der zweiten Konfiguration des Aufstellelementes die Membranschicht in dem Aufnahmebereich die Folienschicht bedeckt, kann dann also der Probenträger in der zweiten Konfiguration einem Stanzvorgang in der beschreibenen Art und Weise zugeführt werden.

Vorgeschlagen wird ferner ein Verfahren zur Gewinnung eines Membranelementes mit getrocknetem Blutbestandteil. In einem ersten Schritt wird ein erfindungsgemäßer Probenträger bereitgestellt. Dieser Probenträger wird auf einen Auflagebereich aufgelegt. Ein Gefäß wird unterhalb des Auflagebereiches bereitgestellt. Es wird dann ein Membranelement aus dem Aufnahmebereich des Probenträgers mittels einer Stanzvorrichtung bzw. mittels eines Stanzmessers ausgestanzt, sodass das ausgestanzte Membranelement und ein aus der Folienschicht ausgestanztes Folienelement in das Gefäß hineinfallen.

Vorzugsweise weist das Verfahren ferner die Schritte auf
- Kontaktieren des Membranelementes in dem Gefäß mit einer Flüssigkeit, die geeignet ist, um den Analyten aus dem getrockneten Blutbestandteil aufzunehmen und
- Nachweisen des Analyten in der Flüssigkeit.

Vorgeschlagen wird ferner eine Verwendung eines erfindungsgemäßen Probenträgers. Dieses kann erfolgen zur qualitativen Bestimmung eines Vorhandenseins eines Analyten in dem getrockneten Blutbestandteil. Alternativ kann die Verwendung erfolgen zur semi-quantitativen Bestimmung einer Konzentration eines Analyten in dem getrockneten Blutbestandteil. Vorzugsweise kann die Verwendung erfolgen zur quantitativen Bestimmung einer Konzentration eines Analyten in dem getrockneten Blutbestandteil.

Im Folgenden wird die Erfindung anhand spezieller Ausführungsformen ohne Beschränkung des allgemeinen Erfindungsgedankens anhand der Figuren näher erläutert. Dabei zeigen:
Figur 1 eine Aufsicht auf eine bevorzugte Ausführungsform des erfindungsgemäßen Probenträgers,
Figur 2 eine Seitenansicht auf die bevorzugte Ausführungsform des erfindungsgemäßen Probenträgers,
Figur 3 eine weitere Ausführungsform des erfindungsgemäßen Probenträgers,
Figuren 4a und 4b die weitere Ausführungsform des erfindungsgemäßen Probenträgers in unterschiedlichen Konfigurationen,
Figur 5 eine erste Konstellation während eines Stanzvorgangs zum Ausstanzen eines Membranelementes,
Figur 6 eine zweite Konstellation während eines Stanzvorgangs zum Ausstanzen eines Membranelementes,
Figur 7 und Figur 8 jeweilige Probenträger, welche jeweils eine Membranschicht und jeweils eine Folienschicht aufweisen,
Figur 9 eine erste Darstellung von Experimentalergebnissen in Prozentzahlen,
Figur 10 eine Darstellung von Experimentalergebnissen mit Absolutzahlen und Prozentzahlen.

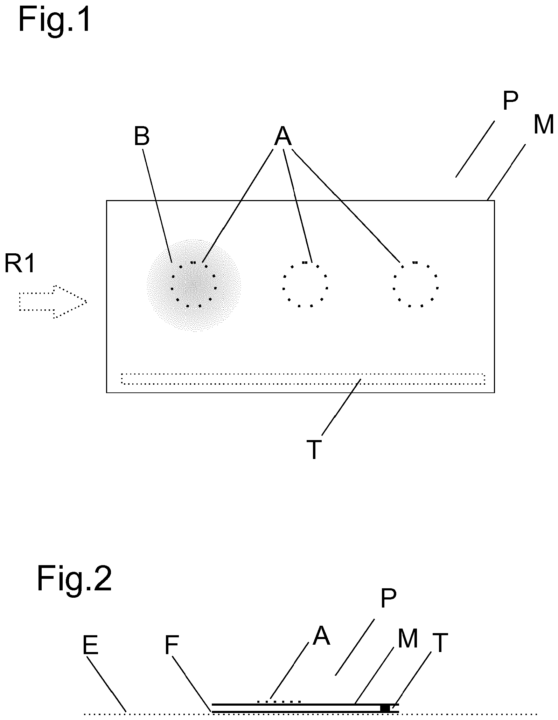

Figur 1 zeigt einen Probenträger P mit einer Membranschicht M, welche auf einem gehobenen Polyolefin basiert. Die Membranschicht M weist wenigstens einen oder mehrere Aufnahmebereiche A auf, wobei wenigstens einer der Aufnahmebereiche A einen getrockneten Blutbestandteil B aufweist. Dieser Blutbestandteil kann Vollblut, insbesondere Kapillarblut, oder Blutplasma oder Blutserum sein.

Ein Teilbereich T, vorzugsweise ein Randbereich, ist gestrichelt gekennzeichnet.

Die Figur 1 zeigt ferner einen Pfeil R1, welcher eine Seitenansichtsperspektive indiziert, aus welcher der Probenträger P in der Figur 2 dargestellt ist.

Die Figur 2 zeigt den Probenträger P mit der Membranschicht M und einem Aufnahmebereich A. Die Blutbestandteile B aus der Figur 1 sind nicht explizit eingezeichnet.

Unterhalb der Membranschicht M befindet sich eine Folienschicht F.

Der Probenträger P ist auf einer ebenen Fläche E aufgelegt.

Die Folienschicht F basiert auf einem Kunststoff, wobei die Folienschicht F auf wenigstens einer Seite, bevorzugt sowohl auf der Oberseite als auch auf der Unterseite der Folienschicht F, antistatisch vorbehandelt ist.

In dem Teilbereich T sind die Membranschicht M und die Folienschicht F miteinander mechanisch verbunden. Dieses kann beispielsweise durch einen Klebestreifen in dem Teilbereich T gegeben sein.

In dem Aufnahmebereich A sind die Folienschicht F und die Membranschicht M nicht miteinander mechanisch verbunden.

Bei Auflegen des Probenträgers P auf die ebene Fläche E bedeckt die Membranschicht M die Folienschicht F zumindest in dem Aufnahmebereich A.

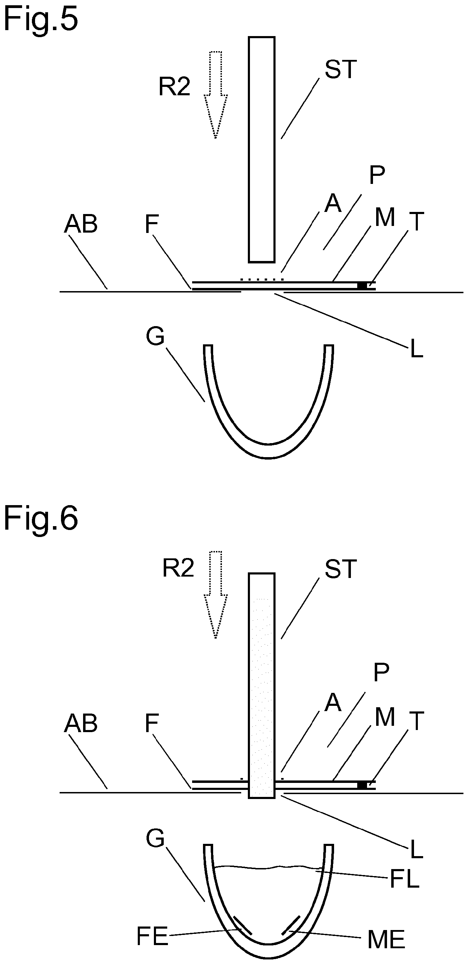

Die Figur 5 zeigt den Probenträger P aus der Figur 2 und der Figur 1 in der Seitenansicht aus der Figur 2 in dem Fall, dass der Probenträger P auf einen Auflagebereich AB aufgelegt ist. Der Auflagebereich AB weist vorzugsweise eine Lochöffnung L auf.

Unterhalb des Auflagebereiches AB ist ein Gefäß G bereitgestellt.

Mittels einer Stanzvorrichtung ST wird aus der Membranschicht M ein Membranelement aus dem Aufnahmebereich A des Probenträgers P ausgestanzt, sodass das ausgestanzte Membranelement dann zusammen mit einem aus der Folienschicht F ausgestanzten Folienelement in das Gefäß G hineinfallen soll. Hierzu wird die Stanzvorrichtung bzw. das Stanzmesser ST von oben her in einer senkrecht zu dem Auflagebereich AB verlaufenden Richtung R2 geführt.

Figur 6 zeigt eine weitere Konstellation, in welcher das Stanzmesser bzw. die Stanzvorrichtung ST ein Membranelement ME als auch ein Folienelement FE aus dem Probenträger P ausgestanzt hat. Ein erfolgreiches Stanzen liegt dann vor, wenn sowohl das Membranelement ME als auch das Folienelement FE in das Gefäß hineinfallen.

Vorzugsweise kann dann das Membranelement ME in dem Gefäß G mit einer biochemischen Flüssigkeit bzw. einer Reagenzflüssigkeit FL in Kontakt gebracht werden, um ein Vorhandensein eines Analyten oder aber einer semiquantitative oder alternativ eine quantitative Bestimmung einer Konzentration eines Analyten in dem getrockneten Blutbestandteil zu bestimmen.

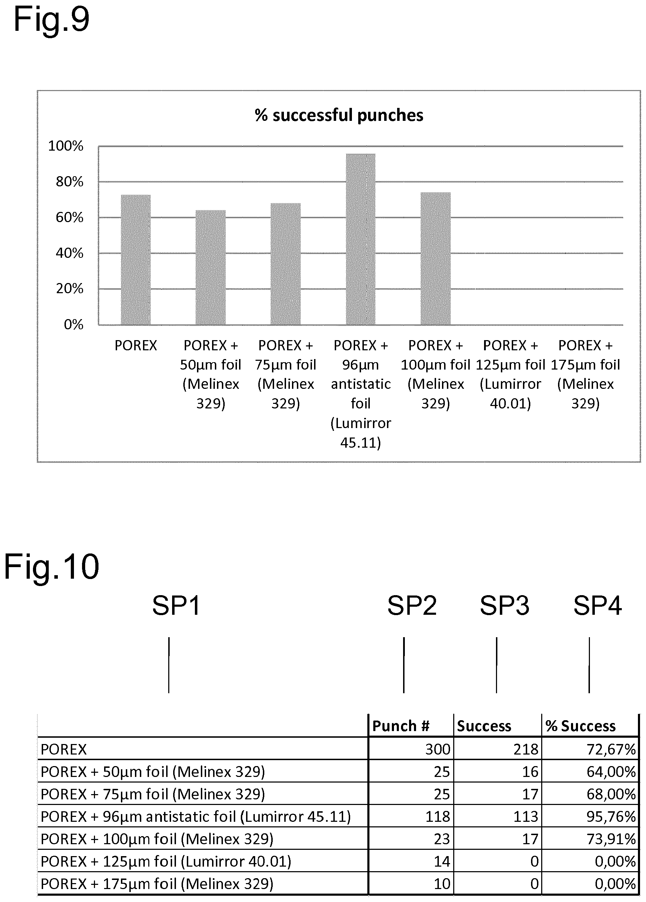

Figur 9 zeigt prozentuale Auswertungsergebnisse von Stanzversuchen, bei welchen als Stanzvorrichtung die Vorrichtung Panthera Puncher 9 des Herstellers PerkinElmer verwendet wurde. Die Membranschicht des Probenträgers basiert auf einem nicht gewobenen Polyolefin und ist hier eine Membran des Typs Porex BNW441435.

Ein Stanzvorgang wurde dann als erfolgreich beurteilt, wenn sowohl das Membranelement als auch das Folienelement in das dafür bereitgestellte Gefäß hineinfielen. Als Gefäß wurden Vertiefungen ('wells') von 96er Mikrotiterplatten deep-well Platten aus Polypropylen verwendet, Modell Eppendorf 30501306. Alternativ können aber auch Standard-Mikrotiterplatten mit geringerer Höhe und aus anderem Material, vorzugsweise Polystyrol, verwendet werden.

Fehlerfälle konnten sich in der Weise ergeben, dass:
- das Membranelement aufgrund zu hohen mechanischen Widerstandes durch eine unterliegende Folienschicht nicht ausgestanzt werden konnte, oder
- das Membranelement und das Folienelement zwar ausgestanzt wurden aber nicht in das unmittelbar unterhalb positionierte Gefäß hineinfielen sondern daneben, oder
- zwar das ausgestanzte Membranelement in dem bereitgestellten Gefäß aufgenommen wurde aber das ausgestanzte Folienelement nicht in das Gefäß gelangte, sondern in ein daneben positioniertes Gefäß einer Mikrotiterplatte, welches eine Kreuzkontamination der Proben untereinander bewirken kann.

Die Figur 10 zeigt zu den prozentualen Auswertungsergebnissen der Figur 9 die zugehörigen Absolutwerte. In der ersten Spalte SP1 sind die jeweiligen Konfigurationen aus Membranschicht (POREX) und zugehörigen Folienschichten aufgeführt. Hierbei befinden sich in den jeweiligen Einträgen der Spalte 1 auch die genauen Produktbezeichnungen der entsprechenden Folienschichten mit ihren jeweiligen Dicken in Mikrometern. Herauszuheben ist, dass nur die Folie Lumirror 45.11 eine antistatisch beschichtete Folie ist.

In der Spalte SP2 ist die Anzahl der durchgeführten Stanzversuche aufgeführt. In der Spalte SP3 ist die absolute Anzahl erfolgreich durchgeführter Stanzversuche aufgeführt. In der Spalte SP4 sind die jeweiligen prozentualen Anteile erfolgreich durchgeführter Stanzversuche aufgeführt.

Für den Fall, dass keinerlei Folie bzw. keine Folienschicht verwendet wurde, ergibt sich bei Verwendung nur einer POREX-Membran mit Membranschicht ohne Folienschicht eine erfolgreiche Stanzrate von 72,67 %.

Werden unterschiedliche Folien verwendet, welche nicht antistatisch vorbehandelt sind, so ergeben sich erfolgreiche Stanzversuche mit einem Anteil, welche unterhalb von 75 % liegen. In der Ausführungsform einer Folienschicht mit einer Dicke von 125 µm oder 175 µm wurden gar keine Stanzversuche erfolgreich durchgeführt, da aufgrund der mechanischen Eigenschaften dieser Folien ein Stanzen nicht erfolgreich durchgeführt werden konnte und Membranelemente gar nicht aus gestanzt wurden.

Bei Verwendung der antistatisch vorbehandelten Folie Lumirror 45.11 mit einer Dicke von 96 µm ergab sich eine deutlich erhöhte erfolgreiche Rate an Stanzversuchen, hier mit einem erfolgreichen Anteil von 95,76 %.

Es wird deutlich, dass eine antistatische Vorbehandlung der Folienschicht deutlich verbesserte Ergebnisse erzielt.

Die hier verwendete Folienschicht des Typs Lumirror 45.11 ist antistatisch vorbehandelt und ist eine Folienschicht, welche auf Polyester basiert.

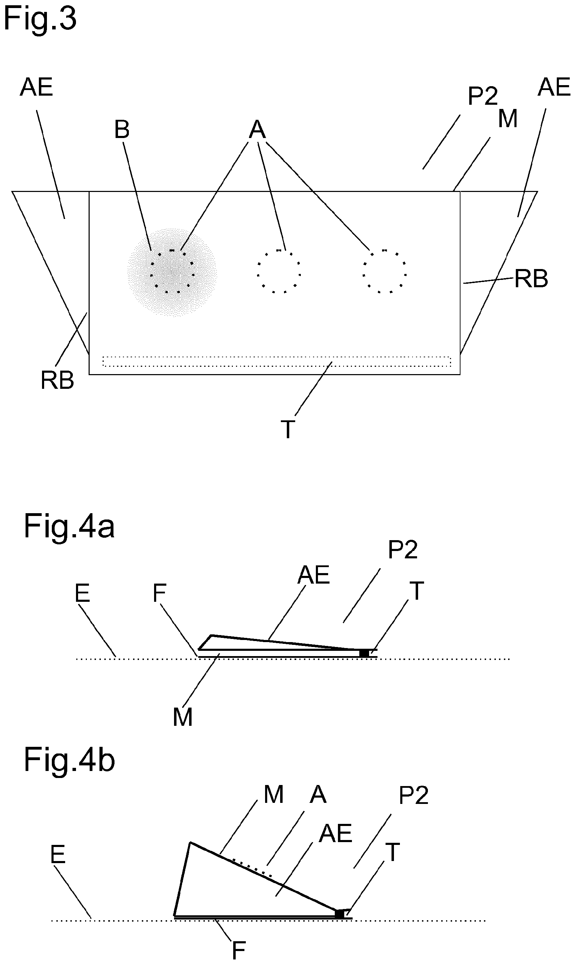

Die Figur 3 zeigt eine weitere bevorzugte Ausführungsform P2 des erfindungsgemäßen Probenträgers. Im Vergleich zu der ersten Ausführungsform des erfindungsgemäßen Probenträgers P aus der Figur 1 weist diese Ausführungsform P2 des Probenträgers ferner ein oder mehrere Aufstellelemente AE auf. Ein Aufstellelement AE ist vorzugsweise durch ein Papp- oder ein Papierelement gegeben, welches an einem Randbereich RB der Membranschicht M befestigt ist. Das Aufstellelement AE kann entlang des Randbereichs bzw. einer entsprechenden Knickkante gefaltet und so ausgerichtet werden. Die Figur 3 zeigt den Probenträger P2 in einer sogenannten zweiten Konfiguration, in welcher er auf eine ebene Fläche aufgelegt ist, sodass die Membranschicht M in dem Aufnahmebereich A die Folienschicht F bedeckt.

Die Figur 4a zeigt den Probenträger P2 in dieser sogenannten zweiten Konfiguration, wobei die Aufstellelemente AE in dieser Seitenansicht leicht nach oben gebogen sind, sodass die Membranschicht M die Folienschicht F in dem aus der Figur 2 angezeichneten Aufnahmebereich A, welcher hier nicht direkt ersichtlich ist, bedeckt.

Die Figur 4b zeigt den Probenträger P2 in einer ersten Konfiguration, in welcher das Aufstellelement AE so aus der Konfiguration der Figur 3 nach unten hinweg geknickt ist, dass ein Aufstellen in der Weise erreicht werden kann, sodass die Membranschicht M von der Folienschicht F beabstandet ist, wenn der Probenträger P2 auch an der ebenen Fläche E aufgelegt ist.

Hierdurch wird es ermöglicht, dass nach Aufbringen eines bestimmten Blutbestandteils auf den Aufnahmebereich A der Membranschicht M dieser Blutbestandteil in die Membranschicht M eintrocknen kann, ohne dass die Folienschicht F eine Unterseite der Membranschicht M berührt. Hierdurch wird es vermieden, dass Teile eines Blutbestandteils die Membranschicht M verlassen und auf einer Oberfläche der Folienschicht F antrocknen.

Die Aufstellelemente AE sind also vorzugsweise Faltelemente an den Seiten des Probenträgers P2, sodass der Probenträger P2 in der Weise durch die Faltelemente AE gestützt wird, dass in der ersten Konfiguration die Membranschicht M in dem Bereich des Aufnahmebereiches A von der Folienschicht beabstandet ist.

In der ersten Konfiguration wird durch das Aufstellelement AE oder die Aufstellelemente AE bei Auflegen des Probenträgers P2 auf die ebene Fläche E eine Beabstandung der Folienschicht F von der Membranschicht M bewirkt, sodass die Folienschicht F auf der ebenen Fläche E zum Aufliegen kommt.

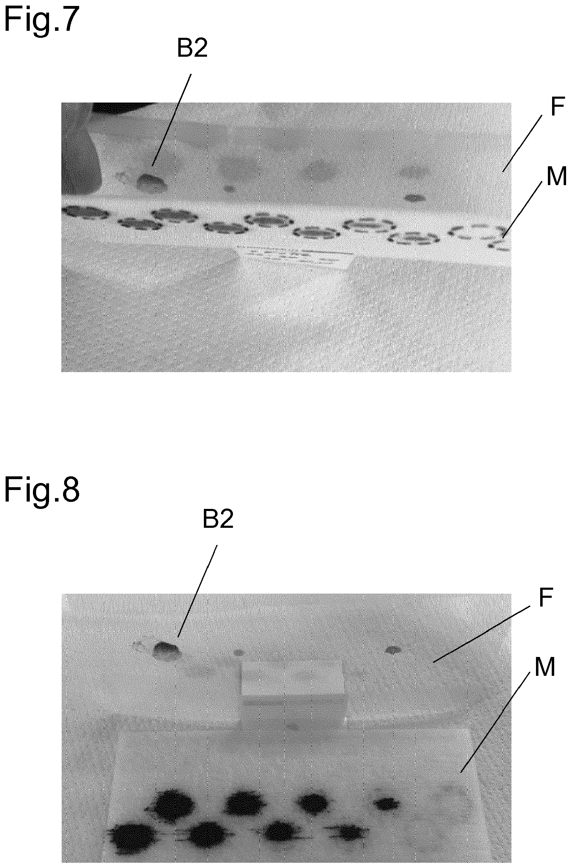

Die Figuren 7 und 8 zeigen hierbei jeweilige Membranschichten M und Folienschichten F, bei welchen aufgrund einer Kontaktierung der Folienschicht F mit der Membranschicht M während des Trocknungsvorganges Teilmengen B2 von Blutbestandteilen auf einer jeweiligen Oberfläche der jeweiligen Folienschichten F angetrocknet sind. Dieses ist eben zu vermeiden und wird durch die Ausführungsform des Probenträgers P2 aus den Figuren 3, 4a und 4b erreicht.

## Patentansprüche

1. Probenträger (P, P2) aufweisend
- eine absorptionsfähige Membranschicht (M), welche auf einem nicht gewobenen Polyolefin basiert und welche wenigstens einen Aufnahmebereich (A) mit getrockneten Blutbestandteilen (B) aufweist,
- sowie ferner eine Folienschicht (F) basierend auf einem Kunststoff, wobei die Folienschicht antistatisch vorbehandelt ist,
wobei die Membranschicht (M) und die Folienschicht (F) in einem Teilbereich (T) des Probenträgers miteinander mechanisch verbunden sind,
wobei ferner die Membranschicht und die Folienschicht in dem Aufnahmebereich (A) nicht miteinander mechanisch verbunden sind
und wobei bei Auflegen des Probenträgers (P, P2) auf eine ebene Fläche (E) zumindest in dem Aufnahmebereich (A) die Membranschicht (M) die Folienschicht (F) bedeckt.

2. Probenträger nach Anspruch 1,
wobei der Kunststoff ein Polyester ist.

3. Probenträger nach Anspruch 2,
wobei der Polyester Polyethylenterephthalat (PET) ist.

4. Probenträger nach einem der vorhergehenden Ansprüche,
wobei die Folienschicht (F) in dem Sinne antistatisch vorbehandelt ist, dass die Folienschicht zumindest in einem Teilbereich ihrer Oberfläche ein Antistatikum aufweist.

5. Probenträger nach einem der vorhergehenden Ansprüche,
wobei das Polyolefin ausgewählt ist aus einer Gruppe umfassend Polyethylen, Polypropylen, Polymethylpente, Polyisobutylen und Polybutylen.

6. Probenträger nach Anspruch 5,
wobei das Polyolefin Polypropylen ist.

7. Probenträger nach einem der vorhergehenden Ansprüche,
wobei der Probenträger (P2) wenigstens ein verstellbares Aufstellelement (AE) aufweist, welches zwischen einer ersten Konfiguration und einer zweiten Konfiguration verstellbar ist,
wobei in der ersten Konfiguration das Aufstellelement (AE) bei Auflegen des Probenträgers (P2) auf die ebene Fläche eine Beabstandung der Membranschicht von der Folienschicht ermöglicht
und wobei in der zweiten Konfiguration bei Auflegen des Probenträgers (P2) auf eine ebene Fläche (E) die Membranschicht (M) zumindest in dem Aufnahmebereich (A) die Folienschicht (F) bedeckt.

8. Verfahren zur Gewinnung eines Membranelementes mit getrockneten Blutbestandteilen aufweisend
- Bereitstellen eines Probenträgers (P, P2) nach einem der Ansprüche 1 bis 7,
- Auflegen des Probenträgers (P, P2) auf einen Auflagebereich (AB),
- Bereitstellen eines Gefäßes (G) unterhalb des Auflagebereiches (AB),
- Ausstanzen des Membranelementes (ME) aus dem Aufnahmebereich (A) des Probenträgers mittels einer Stanzvorrichtung (ST), so dass das ausgestanzte Membranelement (ME) und ein aus der Folienschicht (F) ausgestanztes Folienelement (FE) in das Gefäß (G) fallen.

9. Verfahren nach Anspruch 8,
ferner aufweisend
- Kontaktieren des Membranelementes (ME) in dem Gefäß (G) mit einer Flüssigkeit (FL), die geeignet ist, um den Analyten aus dem getrockneten Blutbestandteil aufzunehmen und
- Nachweisen des Analyten in der Flüssigkeit.

10. Verwendung eines Probenträgers nach einem der Ansprüche 1 bis 7
- zur qualitativen Bestimmung eines Vorhandenseins eines Analyten in dem getrockneten Blutbestandteil,
- zur semi-quantitativen Bestimmung einer Konzentration eines Analyten in dem getrockneten Blutbestandteil,
- oder zur quantitativen Bestimmung einer Konzentration eines Analyten in dem getrockneten Blutbestandteil.

## Claims

1. Sample support (P, P2) having
- an absorbent membrane layer (M) which is based on a nonwoven polyolefin and which has at least one take-up region (A) with dried blood constituents (B),
- and also moreover a film layer (F) based on a plastic, where the film layer has received antistatic pretreatment,
wherein the membrane layer (M) and the film layer (F) are connected to one another mechanically in a subregion (T) of the sample support,
wherein furthermore the membrane layer and the film layer are not connected to one another mechanically in the take-up region (A)
and wherein, when the sample support (P, P2) is placed onto a uniformly horizontal surface (E) the membrane layer (M) covers the film layer (F) at least in the take-up region (A).

2. Sample support according to Claim 1,
wherein the plastic is a polyester.

3. Sample support according to Claim 2,
wherein the polyester is polyethylene terephthalate (PET).

4. Sample support according to any of the preceding claims,
wherein the film layer (F) has received antistatic pretreatment such that the film layer has an antistatic agent at least in a subregion of its surface.

5. Sample support according to any of the preceding claims,
wherein the polyolefin is selected from a group comprising polyethylene, polypropylene, polymethylpentene, polyisobutylene and polybutylene.

6. Sample support according to Claim 5,
wherein the polyolefin is polypropylene.

7. Sample support according to any of the preceding claims,
wherein the sample support (P2) comprises at least one adjustable erection element (AE) which can be adjusted between a first configuration and a second configuration,
wherein in the first configuration, when the sample support (P2) is placed onto the uniformly level surface, the erection element (AE) permits distancing of the membrane layer from the film layer
and wherein in the second configuration, when the sample support (P2) is placed onto a uniformly level surface (E), the membrane layer (M) covers the film layer (F) at least in the take-up region (A).

8. Process for obtaining a membrane element with dried blood constituents comprising
- Provision of a sample support (P, P2) according to any of Claims 1 to 7,
- Placement of the sample support (P, P2) onto a placement region (AB),
- Provision of a vessel (G) below the placement region (AB),
- Punching-out of the membrane element (ME) from the take-up region (A) of the sample support by means of a punching device (ST), in a manner that causes the punched-out membrane element (ME) and a film element (FE) punched out from the film layer (F) to fall into the vessel (G).

9. Process according to Claim 8,
moreover comprising
- Bringing the membrane element (ME) in the vessel (G) into contact with a liquid (FL) that is suitable for take-up of the analyte from the dried blood constituent and
- Detection of the analyte in the liquid.

10. Use of a sample support according to any of Claims 1 to 7
- for the qualitative determination of presence of an analyte in the dried blood constituent,
- for the semi-quantitative determination of a concentration of an analyte in the dried blood constituent,
- or for the quantitative determination of a concentration of an analyte in the dried blood constituent.

## Revendications

1. Support d'échantillon (P, P2) comprenant :
- une couche de membrane apte à l'absorption (M), qui est à base d'une polyoléfine non tissée et qui comprend au moins une zone d'absorption (A) munie de constituants sanguins séchés (B),
- ainsi qu'également une couche de feuille (F) à base d'une matière plastique, la couche de feuille étant prétraitée de manière antistatique,
dans lequel la couche de membrane (M) et la couche de feuille (F) sont reliées mécaniquement l'une à l'autre dans une zone partielle (T) du support d'échantillon, dans lequel en outre la couche de membrane et la couche de feuille ne sont pas reliées mécaniquement l'une à l'autre dans la zone d'absorption (A),
et dans lequel, lors de la pose du support d'échantillon (P, P2) sur une surface plane (E), la couche de membrane (M) recouvre la couche de feuille (F) au moins dans la zone d'absorption (A).

2. Support d'échantillon selon la revendication 1,
dans lequel la matière plastique est un polyester.

3. Support d'échantillon selon la revendication 2,
dans lequel le polyester est le polyéthylène téréphtalate (PET).

4. Support d'échantillon selon l'une quelconque des revendications précédentes,
dans lequel la couche de feuille (F) est prétraitée de manière antistatique au sens que la couche de feuille comprend un antistatique au moins dans une zone partielle de sa surface.

5. Support d'échantillon selon l'une quelconque des revendications précédentes,
dans lequel la polyoléfine est choisie dans un groupe comprenant le polyéthylène, le polypropylène, le polyméthylpentène, le polyisobutylène et le polybutylène.

6. Support d'échantillon selon la revendication 5,
dans lequel la polyoléfine est le polypropylène.

7. Support d'échantillon selon l'une quelconque des revendications précédentes, dans lequel le support d'échantillon (P2) comprend au moins un élément de soulèvement ajustable (AE), qui est ajustable entre une première configuration et une deuxième configuration,
dans lequel, dans la première configuration, l'élément de soulèvement (AE) permet, lors de la pose du support d'échantillon (P2) sur la surface plane, un espacement de la couche de membrane de la couche de feuille,
et dans lequel, dans la deuxième configuration, lors de la pose du support d'échantillon (P2) sur une surface plane (E), la couche de membrane (M) recouvre la couche de feuille (F) au moins dans la zone d'absorption (A).

8. Procédé d'obtention d'un élément de membrane muni de constituants sanguins séchés, comprenant :
- la fourniture d'un support d'échantillon (P, P2) selon l'une quelconque des revendications 1 à 7,
- la pose du support d'échantillon (P, P2) sur une zone de pose (AB),
- la fourniture d'un récipient (G) en dessous de la zone de pose (AB),
- le poinçonnage de l'élément de membrane (ME) à partir de la zone d'absorption (A) du support d'échantillon au moyen d'un dispositif de poinçonnage (ST), de telle sorte que l'élément de membrane poinçonné (ME) et un élément de feuille (FE) poinçonné à partir de la couche de feuille (F) tombent dans le récipient (G).

9. Procédé selon la revendication 8,
comprenant en outre :
- la mise en contact de l'élément de membrane (ME) dans le récipient (G) avec un liquide (FL), qui est approprié pour absorber l'analyte à partir du constituant sanguin séché, et
- la détection de l'analyte dans le liquide.

10. Utilisation d'un support d'échantillon selon l'une quelconque des revendications 1 à 7
- pour la détermination qualitative d'une présence d'un analyte dans le constituant sanguin séché,
- pour la détermination semi-quantitative d'une concentration d'un analyte dans le constituant sanguin séché,
- ou pour la détermination quantitative d'une concentration d'un analyte dans le constituant sanguin séché.
